# FASCICULE DE BREVET EUROPEEN

(11) **EP 0 973 467 B1**
(45) Date de publication et mention de la délivrance du brevet: **19.02.2003**
(21) Numéro de dépôt: 98917257.2
(22) Date de dépôt: 27.03.1998
(51) Int. Cl.: A61F 11/00

(54) **DISPOSITIF DE NETTOYAGE ET D'EXTRACTION DE CERUMEN**
VORRICHTUNG ZUR GEHÖRGANGREINIGUNG UND ZUR ENTFERNUNG VON OHRENSCHMALZ
DEVICE FOR CLEANING AND REMOVING EARWAX

(30) Priorité: 28.03.1997 FR 9703823
(43) Date de publication de la demande: 26.01.2000
(73) Titulaire: Capital Innovation S.A.R.L., 93406 Saint-Ouen (FR); Beuttler, Laurent, 49000 Angers (FR); Optos-Opus S.A.R.L., 75015 Paris (FR); Corbin, Jean-Yves, 75012 Paris (FR); Contencin, Philippe, 92200 Neuilly-sur-Seine (FR); Groupe PBE S.A., 93406 Saint-Ouen (FR); TENAILLE D'ESTAIS, Mathias, 75012 Paris (FR)
(72) Inventeur: TENAILLE D'ESTAIS, Mathias, F-75012 Paris (FR)
(74) Mandataire: Benech, Frédéric
(86) Numéro de dépôt international: FR9800629
(87) Numéro de publication internationale: WO98043564

(56) Documents cités:
- EP-A- 0 744 168
- WO-A-96/37172
- US-A- 2 331 732
- US-A- 5 632 756

## Description

La présente invention concerne un dispositif pour le nettoyage et l'extraction de produit cireux accumulé dans un conduit auditif, comprenant une tige de préhension du dispositif par un utilisateur, allongée autour d'un axe et munie d'au moins une portion d'extrémité en saillie radialement par rapport à la tige.

Elle trouve une application particulièrement importante, bien que non exclusive, dans le domaine de l'hygiène et de l'esthétique permettant d'éviter l'accumulation de cérumen.

Le cérumen est une substance de consistance molle, de PH acide, d'aspect cireux qui, lorsqu'il vieillit et qu'il s'oxyde à l'air, change de couleur et perd de sa fluidité. Il peut alors boucher les conduits auditifs et générer des surdités et/ou pertes d'équilibre.

Traditionnellement, on a donc cherché à l'éliminer et c'est ainsi que sont apparus les cotons-tiges.

Ceux-ci présentent cependant des inconvénients.

En effet, le coton-tige a tendance à repousser le cérumen et à provoquer l'apparition de bouchon dans le conduit auditif.

De plus, d'une part il irrite le conduit auditif externe et d'autre part il peut causer certains accidents comme des tympans abîmés ou crevés.

Compte tenu de ces inconvénients, d'autres solutions ont été proposées.

Le document FR-A-2 606 883 décrit par exemple un dispositif d'évacuation complète du cérumen par l'intermédiaire d'un élément extracteur muni de griffes déformables.

Un tel dispositif risque néanmoins de repousser le bouchon au fond du conduit plutôt que de l'extraire.

D'autres dispositifs existent, plus particulièrement pratiqués par des médecins, constitués par exemple par des raclettes, comme l'anneau de Trautmann ou l'ance de Snellen, permettant de retirer le cérumen.

Ces dispositifs sont d'une utilisation délicate, demandant l'intervention d'un spécialiste afin d'éviter les traumatismes.

Ils nécessitent de plus une stérilisation à chaque intervention.

On connaît également (EP 0 744 168) un dispositif comprenant une portion d'extrémité en forme de cage pour racler le conduit auditif et muni d'une pièce buttoir pour nettoyer simultanément l'entrée du conduit, ou encore (WO-96/37172) avec branches de section trapézoïdale.

Le document WO-96/37172 constitue le préambule de la revendication 1.

De tels dispositifs sont coûteux, peu agréables à utiliser et ne permettent pas de tenir compte des longueurs et largeurs différentes de conduits auditifs.

La présente invention vise à fournir un dispositif pour le nettoyage et l'extraction de produit cireux accumulé dans un conduit auditif, répondant mieux que ceux antérieurement connus aux exigences de la pratique, notamment en ce qu'elle permet une extraction non agressive tenant compte des différents types d'oreilles, en ce qu'elle évite de repousser le cérumen lors de l'introduction dans le conduit, et ce sans risquer d'irriter et/ou de blesser le conduit auditif.

Par ailleurs, le dispositif proposé est d'une utilisation facile et agréable, présente un faible coût de fabrication et est de ce fait jetable, ce qui permet de respecter une bonne hygiène en évitant le développement de microbes.

Avec le dispositif selon l'invention, il va par ailleurs être possible d'enlever une bonne partie du cérumen tout en laissant une couche de protection.

On sait en effet que le cérumen est une substance de consistance molle et, que par son PH acide et sa nature cireuse, il protège également le revêtement cutané du conduit auditif.

Il a une action anti-bactérienne et antifongique liée au PH acide et à la présence de lyposomes.

La pellicule de cire constitue donc non seulement une barrière chimique, mais également un véritable piège à corps étranger. Il est donc intéressant d'enlever la partie en excédant, mais de laisser une mince pellicule qui va permettre la protection naturelle comme indiqué ci-dessus.

Dans ce but l'invention propose notamment un dispositif pour le nettoyage et l'extraction de produits cireux accumulés dans un conduit auditif selon la revendication 1. Ce dispositif comprend une tige de préhension par un utilisateur, allongée autour d'un axe et munie d'au moins une portion d'extrémité en saillie radialement par rapport à ladite tige,
la portion d'extrémité étant en forme de cage de section longitudinale sensiblement ovale comportant au moins trois branches courbes réparties angulairement et reliées entre elles de chaque côté par leurs extrémités respectives pour constituer au moins trois anses fermées rattachées par leur sommets, et propres à racler le conduit auditif lors d'un mouvement tournant de la tige effectué par l'utilisateur, caractérisé en ce que
ladite portion d'extrémité présente un sommet de raccordement des branches entre elles par une portion de surface comprise entre 1 et 3 mm², lesdites branches étant de faible épaisseur et de section transversale polygonale, ladite portion d'extrémité étant souple du côté du sommet, de sorte qu'elle peut se déformer élastiquement radialement sous la pression normale manuelle d'un utilisateur, et rigide dans la partie la plus proche de la tige.

Par faible épaisseur, il faut entendre une épaisseur dans le sens tangentiel à la cage de dimension inférieure à de l'ordre de 2 mm, et plus particulièrement inférieure à 1 mm, par exemple 0,5 mm.

Le sommet de la cage de forme ovoïde, présente ainsi une calotte ajourée sur trois, quatre secteurs ou plus, les branches étant raccordées entre elles par une portion de faible surface, la faible épaisseur des branches et la portion de faible surface permettant d'éviter de repousser le cérumen vers le tympan comme le coton-tige classique.

Par portion d'extrémité souple du côté du sommet il faut.entendre une cage susceptible de se déformer élastiquement radialement sous la pression normale manuelle d'un utilisateur par exemple de 10 à 50 %, par exemple d'une valeur de 20 %, de la dimension transversale (c'est-à-dire dans le sens radial) de la cage dans sa plus grande section, sous l'effet d'une force longitudinale, c'est-à-dire dans le sens de la tige, comprise entre de l'ordre de 0,1 et de l'ordre de 0,5 Newton, par exemple de l'ordre de 0,2 Newton.

La différence de souplesse entre sommet et base de la cage rend compatible les besoins de sécurité et de résistance du dispositif selon l'invention.

La section transversale polygonale des branches permet notamment un effet de spatule.

Dans des modes de réalisation avantageux on a de plus recours à l'une et/ou à l'autre des dispositions suivantes :
- la tige comporte deux portions d'extrémité identiques, chacune située à un bout de la tige;
- la portion d'extrémité comporte quatre branches réparties régulièrement, constituant quatre anses fermées rattachées par leur sommet;
- la portion d'extrémité comprend deux jeux de deux branches opposées, les deux branches opposées de chaque jeu étant respectivement situées l'une d'un côté et l'autre de l'autre côté d'un plan axial correspondant audit jeu et passant par le centre du sommet de la cage, les plans axiaux respectifs desdits jeux étant perpendiculaires entre eux;
- les branches sont recouvertes d'un produit enducteur de contact doux au toucher;
- le produit enducteur est un matériau fibreux ou cotonneux de faible épaisseur;
- les branches sont de section transversale parallélépipédique variant progressivement d'une valeur comprise entre de l'ordre de 4 mm² et de l'ordre de 0,5 mm² du côté de la tige, à une valeur comprise entre de l'ordre de 1 mm² et de l'ordre de 0,1 mm² du côté du sommet de la cage;
- les branches présentent du côté de leur sommet une section transversale inférieure à de l'ordre de 0,25 mm²;
- la section transversale des branches est rectangulaire;
- la section transversale des branches est trapézoïdale isocèle;
- La section transversale des branches présente une forme polygonale, dont les angles sont arrondis;
- chaque branche comporte une partie en forme de pli située du côté du sommet de la cage et présentant un sommet dirigé vers l'intérieur de la cage;
- la tige est souple;

Par souple, il faut entendre une tige susceptible de prendre une forme arquée, en flexion, avec une flèche par exemple de l'ordre de 1/10ème à 1/5ème de la longueur de la tige sous la pression normale manuelle d'un utilisateur.
- la tige comprend une partie centrale de préhension par les doigts de l'utilisateur, s'étendant de part et d'autre du centre de symétrie de ladite tige, en pente douce et respectivement, vers une excroissance de révolution en saillie, propre à former une butée pour les doigts de l'utilisateur.

L'invention sera mieux comprise à la lecture de la description qui suit des modes de réalisation donnés à titre d'exemples non limitatifs.

La description fait référence aux dessins qui l'accompagnent dans lesquels :
- La figure 1 est une vue schématique du dispositif selon le mode de réalisation de l'invention plus particulièrement décrit ici, utilisé dans un conduit auriculaire humain représenté en coupe.
- La figure 2 est une vue latérale du dispositif de la figure 1.

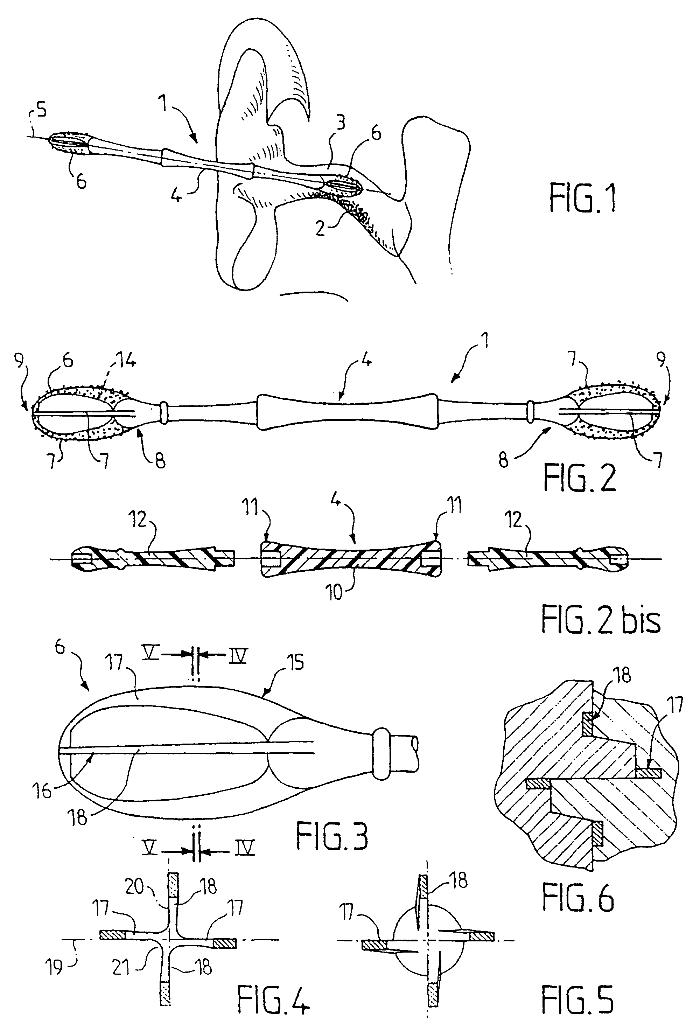

- La figure 2bis est une vue en coupe d'un mode de réalisation de la tige de la figure 2, formée à partir de plusieurs morceaux.
- La figure 3 est une vue à grande échelle de la portion d'extrémité de la figure 2, non enduite.
- Les figures 4 et 5 sont des vues en coupe selon les plans IV-IV et V-V de la figure 3.
- La figure 6 est une vue en coupe, de principe, d'un moule permettant d'obtenir la portion d'extrémité de la figure 3 au niveau de la coupe IV-IV.

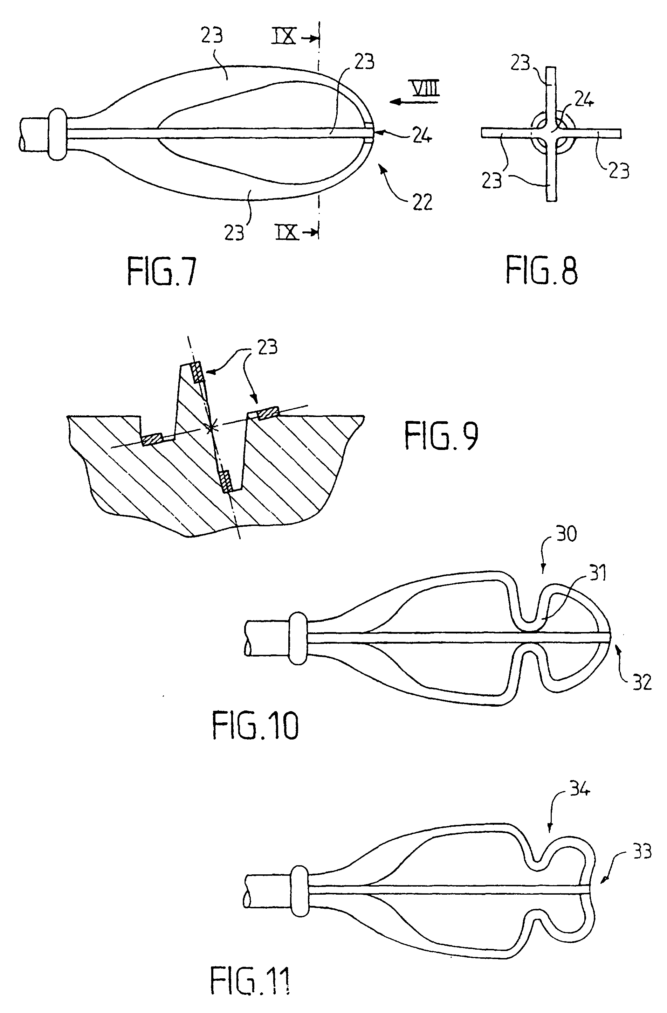

- La figure 7 montre un autre mode de réalisation de la portion d'extrémité du dispositif selon l'invention.
- La figure 8 est une vue de dessus selon la flèche VIII de la portion d'extrémité de la figure 7.
- La figure 9 est une vue en coupe schématique d'un moule permettant d'obtenir la tête de la figure 7, au niveau de la coupe IX-IX.
- Les figures 10 et 11 montrent deux autres modes de réalisation en vue latérale des portions d'extrémité selon l'invention.

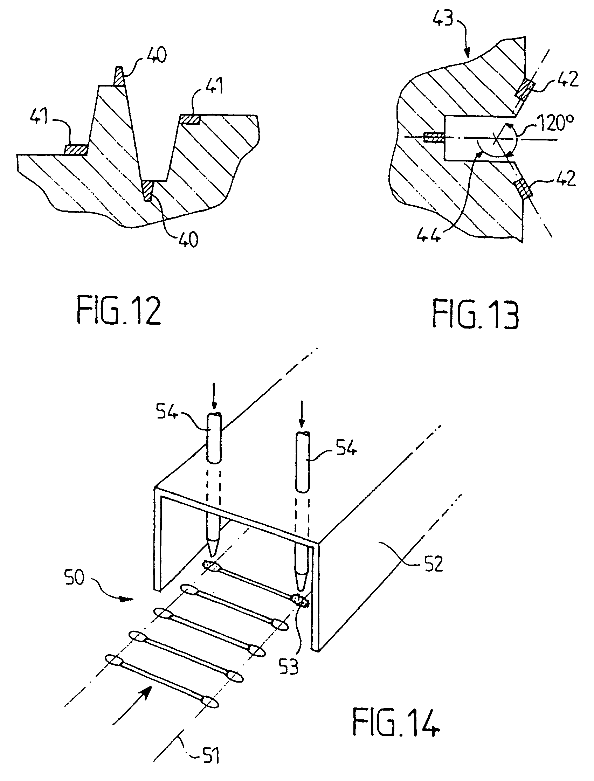

- Les figures 12 et 13 montrent deux autres modes de réalisation de moule, en coupe au niveau des branches de portions d'extrémité selon l'invention.
- La figure 14 est une vue en perspective, schématique, d'un élément d'une chaîne de fabrication des dispositifs selon l'invention, au niveau de l'enduction de protection permettant d'obtenir un contact doux au toucher sur les portions d'extrémité.

La figure 1 montre un dispositif 1 de nettoyage et d'extraction de produit cireux 2 accumulé dans un conduit auditif 3.

Le dispositif 1 comprend une tige de préhension 4, souple, allongée autour d'un axe 5 de révolution, et munie de deux portions d'extrémité 6 en saillie radialement par rapport à la tige 4.

La figure 2 montre le dispositif 1 muni des portions d'extrémité 6, chaque portion d'extrémité 6 forment une cage ovoïde ou de section sensiblement ovale comportant quatre branches identiques 7, courbes, réparties angulairement, à savoir à 90° l'une de l'autre, et raccordées entre elles de chaque côté de leurs extrémités, à savoir du côté 8 de la tige 4, et du côté 9 opposé à la tige.

Les branches courbes forment ainsi quatre anses fermées rattachées par leur sommet présentant une petite surface, par exemple 1,5 mm², qui permettent de racler le conduit auditif.

On a représenté sur la figure 2bis une tige 4 qui comprend une partie centrale 10, de section en forme de papillon présentant à chaque extrémité des parties en saillie il qui vont permettre (Cf. également figure 2) le blocage de l'extrémité des doigts de l'utilisateur en butée, facilitant ainsi la tenue et la préhension du dispositif.

Dans le mode de réalisation de la figure 2bis, les extrémités 12 sont connectables par emboîtement dans des évidements 13 réalisés aux extrémités 11 de la partie centrale 10.

De façon préférée la tige et ses portions d'extrémité vont être réalisées d'une seule pièce, par exemple en polypropylène, dans un moule par injection.

Enfin les branches des extrémités 6 sont recouvertes d'un produit enducteur 14 constitué par un flocage, par exemple en fibre de viscose (cellulose).

Un tel revêtement permet un contact doux au toucher.

On a représenté sur les figures 3, 4 et 5 un mode de réalisation particulièrement avantageux selon l'invention, d'une portion d'extrémité 6, muni de branches 7.

La cage de cette portion d'extrémité comprend deux jeux 15 et 16 identiques de branches opposées 17 et 18, les branches opposées 17 d'un premier jeu, et 18 d'un second jeu étant respectivement situées de part et d'autre d'un plan axial 19, 20 passant par le centre 21 du sommet de la cage (Cf. figure 4).

Les plans axiaux 19 et 20 respectifs sont perpendiculaires.

On a représenté sur la figure 6, en coupe, un principe de moule permettant par un seul mouvement de démoulage d'obtenir les branches de la portion d'extrémité de la figure 3.

Un tel moule particulièrement avantageux du fait de la disposition des branches l'une par rapport à l'autre permet une fabrication simple, rapide et peu coûteuse du dispositif selon l'invention.

On a représenté sur la figure 7 un autre mode de réalisation d'une portion d'extrémité 22 selon l'invention, muni de quatre branches identiques 23, disposées régulièrement et angulairement en vis-à-vis l'une de l'autre comme montré sur la vue de dessus de la figure 8, les deux branches opposées d'un même jeu étant ici situées dans le même plan.

Comme on l'a vu, le sommet de jonction 24 entre les extrémités des branches 23 est d'une surface très réduite, tout en étant de dimension suffisante pour assurer une attache solide entre extrémités.

Une telle disposition évite de. repousser le cérumen lors de l'introduction de la portion d'extrémité dans le conduit auditif.

On a représenté sur la figure 9 le moule en coupe permettant d'obtenir les branches de la portion d'extrémité de la figure 7.

Les branches opposées sont donc disposées dans un même plan, les deux plans des deux jeux de branches étant perpendiculaires.

Avantageusement, la section des branches est rectangulaire et est comprise entre 1 mm² dans leur plus grande dimension du côté de la tige, et 0,2 mm² dans leur plus petite dimension au niveau du sommet de la cage.

L'épaisseur e dans le sens tangentiel est constante par exemple de 0,4 mm, et la largeur l dans le sens radial, varie quant à elle de 2 mm à 0,5 mm.

On a représenté sur les figures 10 et 11 deux autres modes de réalisation de portions d'extrémité selon l'invention.

La figure 10 montre un mode de réalisation où les branches comportent un pli 30, dont le sommet 31 est situé vers l'intérieur de la cage.

Le pli est situé dans le tiers supérieur de la cage du côté du sommet 32, et correspond pratiquement à la largeur de la moitié de la tête.

Il permet d'améliorer la capacité d'amortissement de ladite tête.

La figure 11 montre un autre mode de réalisation où la tête 33 est écrasée par rapport au reste de la portion d'extrémité, un pli 34 étant par ailleurs prévu de façon similaire au pli 30 de la figure 10.

D'autres modes de réalisation des sections des branches peuvent être bien évidemment prévus.

On a ainsi montré sur la figure 12 un moule en coupe au niveau des branches permettant d'obtenir une autre disposition de branches, deux (ou quatre) des branches 40 présentant une section trapézoïdale isocèle, la petite base dirigée vers l'extérieur.

Les deux autres branches 41 peuvent présenter une section rectangulaire.

Dans le cas d'une portion d'extrémité munie de trois branches 42, celles-ci peuvent être formées à partir d'un moule 43 par injection du type représenté sur la figure 13. Dans ce cas les trois branches sont réparties angulairement en formant entre elles des angles 44 de 120°.

On va maintenant décrire la fabrication de dispositifs selon l'invention.

Après formation des tiges et de leur portion d'extrémité d'une seule pièce par injection par exemple, de polypropylène, dans des moules du type décrit ci-avant, les dispositifs 50 (Cf. figure 14) sont amenés sur un tapis roulant 51 en dessous d'un appareil 52 d'injection de flocages au niveau des portions d'extrémité 53 par le biais de buses d'injection 54, avant d'être évacués.

Il est donc obtenu un dispositif souple, notamment au niveau de la tige, ce qui autorise une grande facilité d'utilisation, et permet malgré tout, grâce à une certaine rigidité initiale, une précision importante au niveau des mouvements de la tête du dispositif à partir des actions de l'utilisateur sur le corps du dispositif.

La tige peut se conformer au conduit et permettre une mise en pression optimisée et douce lors du raclage, la section transversale de la partie en saillie étant de préférence comprise entre la moitié et les 3/4 du conduit auditif moyen d'adulte ou d'enfant, afin d'obtenir une bonne mobilité au dispositif.

La section transversale de la partie en saillie peut également être de la largeur du conduit auditif, voire supérieure, et se conformer à celui-ci lorsque la cage du dispositif pénètre dans le conduit du fait de la souplesse de ladite cage.

Le cérumen qui se trouve dans la première moitié du conduit peut être retiré sans éliminer complètement la couche protectrice.

Le caractère doux et éventuellement spongieux du matériau enduit sur les portions d'extrémité permet d'absorber en partie l'eau après une douche ou une piscine.

La disposition des branches permet un auto-centrage de la portion d'extrémité dans le conduit auditif, la dimension de la cage étant par exemple un peu inférieure à celle du conduit auditif.

Plus précisément, la section au niveau du sommet sera la plus fine possible, puis au fur et à mesure qu'on se rapprochera de la tige, l'épaisseur des branches va croître tout en permettant une souplesse de la tête, qui va grossir progressivement afin de permettre une certaine pression sur les parois du conduit.

Les sections des branches, et plus précisément les dimensions radiales des branches continuent à grandir à épaisseur constante jusqu'au manche de la tige ou la rigidité est plus grande, ce qui permet une fixation solide de la portion d'extrémité sur ladite tige.

La souplesse et la finesse de l'extrémité, tout comme la rigidité et la largeur de la partie plus proche de la tige, et la forme parallélépipédique des branches permettent un bon raclage tout en étalant malgré tout en partie de la couche de cérumen restante, avec l'effet protecteur indiqué ci-avant.

Comme il va de soi et comme il résulte d'ailleurs de ce qui précède, la présente invention ne se limite pas aux modes de réalisation plus particulièrement décrits. Elle embrasse au contraire toutes les variantes et notamment celles où le dispositif comporte plus de quatre branches.

## Revendications

1. Dispositif (1) pour le nettoyage et l'extraction de produits cireux (2) accumulés dans un conduit auditif (3), comprenant une tige (4) de préhension du dispositif par un utilisateur, allongée autour d'un axe (5) et munie d'une portion d'extrémité (6) en forme de cage de section longitudinale sensiblement ovale comportant au moins trois branches courbes, réparties angulairement et reliées entre elles de chaque côté par leurs extrémités respectives, lesdites branches (7) étant de section transversale polygonale, **caractérisé en ce que** lesdites branches ont une section qui varie progressivement d'une valeur comprise entre de l'ordre de 4 mm² et de l'ordre de 0,5 mm² du côté de la tige, à une valeur comprise entre de l'ordre de 0,5 mm² et de l'ordre de 0,1 mm² du côté du sommet de la cage, de sorte que ladite portion d'extrémité est souple du côté du sommet, pour se déformer élastiquement radialement sous la pression manuelle d'un utilisateur, et rigide dans la partie la plus proche de la tige, et **en ce que** ladite portion d'extrémité (6) présente un sommet de raccordement des branches entre elles par une portion de surface comprise entre 1 et 3 mm².

2. Dispositif selon la revendication 1, **caractérisé en ce que** la tige (4) comporte deux portions d'extrémité (6) identiques, souples, chacune située à un bout de la tige.

3. Dispositif selon l'une quelconque des revendications précédentes, **caractérisé en ce que** la portion d'extrémité comporte quatre branches (7, 17, 18) réparties régulièrement, constituant quatre anses fermées rattachées par leur sommets.

4. Dispositif selon la revendication 3, **caractérisé en ce que** la portion d'extrémité comprend deux jeux (15, 16) de deux branches (17, 18) opposées, les deux branches opposées (17, 18) de chaque jeu étant respectivement situées l'une d'un côté et l'autre de l'autre côté d'un plan axial (19, 20) correspondant audit jeu et passant par le centre (21) du sommet de la cage, les plans axiaux (19, 20) respectifs desdits jeux étant perpendiculaires entre eux.

5. Dispositif selon l'une quelconque des revendications précédentes, **caractérisé en ce que** les branches (7 ; 17, 18) sont recouvertes d'un produit enducteur (14) de contact doux au toucher.

6. Dispositif selon la revendication 5, **caractérisé en ce que** le produit enducteur (14) est un matériau fibreux ou cotonneux de faible épaisseur.

7. Dispositif selon l'une quelconque des revendications précédentes, **caractérisé en ce que** les branches (7) présentent du côté de leur sommet une section transversale inférieure à de l'ordre de 0,25 mm².

8. Dispositif selon l'une quelconque des revendications précédentes, **caractérisé en ce que** la section transversale des branches (7) est rectangulaire.

9. Dispositif selon l'une quelconque des revendications 1 à 7, **caractérisé en ce que** la section transversale des branches est trapézoïdale isocèle.

10. Dispositif selon l'une quelconque des revendications précédentes, **caractérisé en ce que** chaque branche comporte une partie (30, 34) en forme de pli située du côté du sommet de la cage et présentant un sommet (31) dirigé vers l'intérieur de la cage.

11. Dispositif selon l'une quelconque des revendications précédentes, **caractérisé en ce que** la tige (4) est souple, c'est-à-dire susceptible de prendre une forme arquée, en flexion, avec une flèche de l'ordre d'un dixième à un cinquième de la longueur de la tige sous la pression normale manuelle d'un utilisateur.

12. Dispositif selon l'une quelconque des revendications précédentes, **caractérisé en ce que** la tige (4) comprend une partie centrale (10) de préhension par les doigts de l'utilisateur, s'étendant de part et d'autre du centre de symétrie de ladite tige, en pente douce vers une excroissance (11) de révolution en saillie, propre à former une butée pour les doigts de l'utilisateur.

## Claims

1. Device (1) for the cleaning and extraction of waxy products (2) accumulated in an auditory canal (3), comprising a rod (4) for the gripping of the device by a user, extended about an axis (5) and provided with a cage-shaped end portion (6) with a substantially oval longitudinal section having at least three curved branches, distributed angularly and connected together on each side by their respective ends, said branches (7) being of polygonal cross section, **characterised in that** said branches have a section that varies progressively from a value between about 4 mm² and about 0.5 mm² on the side of the rod, to a value between about 0.5 mm² and about 0.1 mm² on the side of the top of the cage, so that said end portion is flexible on the side of the top, in order to be deformed resiliently radially under manual pressure from a user, and rigid in the part closest to the rod, and **in that** said end portion (6) has a top where the branches are connected together by a surface portion of between 1 and 3 mm².

2. Device according to Claim 1, **characterised in that** the rod (4) has two identical flexible end portions (6), each located at one end of the rod.

3. Device according to any one of the preceding claims, **characterised in that** the end portion has four branches (7, 17, 18) distributed regularly, constituting four closed handles attached by their tops.

4. Device according to Claim 3, **characterised in that** the end portion comprises two sets (15, 16) of two opposite branches (17, 18), the two opposite branches (17, 18) of each set being located respectively one on one side and the other on the other side of an axial plane (19, 20) corresponding to said set and passing through the centre (21) of the top of the cage, the respective axial planes (19, 20) of said sets being perpendicular to each other.

5. Device according to any one of the preceding claims, **characterised in that** the branches (7; 17, 18) are covered with a coating contact product (14) that is soft to the touch.

6. Device according to Claim 5, **characterised in that** the coating product (14) is a fibrous or cottony material of low thickness.

7. Device according to any one of the preceding claims, **characterised in that** the branches (7) have on the side of their top a cross-section less than about 0.25 mm².

8. Device according to any one of the preceding claims, **characterised in that** the cross section of the branches (7) is rectangular.

9. Device according to any one of Claims 1 to 7, **characterised in that** the cross section of the branches is an isosceles trapezoid.

10. Device according to any one of the preceding claims, **characterised in that** each branch has a fold-shaped part (30, 34) located on the side of the top of the cage and having a top (31) pointing towards the inside of the cage.

11. Device according to any one of the preceding claims, **characterised in that** the rod (4) is flexible, that is, capable of taking on an arched shape when bent, with a deflection of the order of one-tenth to one-fifth of the length of the rod under the normal manual pressure of a user.

12. Device according to any one of the preceding claims, **characterised in that** the rod (4) comprises a central part (10) for gripping by the fingers of the user, extending on either side of the centre of symmetry of said rod, gently sloping towards a projecting excrescence (11) generated by rotation, suitable for forming a stop for the fingers of the user.

## Patentansprüche

1. Vorrichtung (1) zur Reinigung und Entfernung von in einem Gehörgang (3) angesammelten Ohrschmalzprodukten (2) mit einem Stab (4) zum Anfassen der Vorrichtung durch einen Benutzer, wobei der Stab (4) sich um eine Achse (5) erstreckt und mit einem Endteil (6) in Form einer Aufnahme mit einem im wesentlichen ovalförmigen Längsquerschnitt versehen ist, das wenigstens drei gekrümmte Zweige aufweist, die winkelmäßig verteilt und an jeder Seite durch ihre jeweiligen Enden miteinander verbunden sind, wobei die Zweige (7) einen vieleckigen Querschnitt aufweisen,
**dadurch gekennzeichnet,**
**dass** die Zweige einen Querschnitt aufweisen, der sich progressiv von einem Wert zwischen ungefähr 4 mm² und ungefähr 0,5 mm² an der Seite des Stabes auf einen Wert zwischen ungefähr 0,5 mm² und ungefähr 0,1 mm² an der Seite der Spitze der Aufnahme ändert, derart, dass das Endteil an der Seite der Spitze nachgiebig ist, um sich unter dem manuellen Druck eines Benutzers elastisch radial zu verformen, und in dem zu dem Stab nächstliegenden Teil starr ist, und dass das Endteil (6) eine Spitze zur Verbindung der Zweige miteinander durch einen Oberflächenteil zwischen 1 und 3 mm² aufweist.

2. Vorrichtung nach Anspruch 1.
**dadurch gekennzeichnet,**
**dass** der Stab (4) zwei identische, nachgiebige Endteile (6) enthält und jedes an einem Ende des Stabes liegt.

3. Vorrichtung nach einem der vorangehenden Ansprüche,
**dadurch gekennzeichnet,**
**dass** das Endteil vier gleichmäßig verteilte Zweige (7, 17, 18) aufweist, die vier über ihre Spitzen mlteinander verbundene geschlossene Bügel bilden.

4. Vorrichtung nach Anspruch 3,
**dadurch gekennzeichnet,**
**dass** das Endteil zwei Zwischenräume (15, 16) von zwei gegenüberliegenden Zwelgen (17, 18) aufweist, wobei die beiden gegenüberliegenden Zweige (17, 18) jedes Zwischenraums jeweils zu beiden Seiten einer axialen Ebene (19, 20) liegen, die dem Zwischenraum entsprechen und durch den Mittelpunkt (21) der Spitze der Aufnahme verlaufen, und die jeweiligen axialen Ebenen (19, 20) der Zwischenräume senkrecht zueinander liegen.

5. Vorrichtung nach einem der vorangehenden Ansprüche.
**dadurch gekennzeichnet,**
**dass** die Zweige (7; 17; 18) durch einen slch weich anfühlenden Belag (14) bedeckt sind.

6. Vorrichtung nach Anspruch 5,
**dadurch gekennzeichnet,**
**dass** der Belag (14) ein faserartiges oder watteartiges Material mit geringer Dicke ist.

7. Vorrichtung nach einem der vorangehenden Ansprüche,
**dadurch gekennzeichnet,**
**dass** die Zweige (7) an der Seite ihrer Spitze einen Querschnitt unterhalb ungefähr 0,25 mm² aufweisen.

8. Vorrichtung nach einem der vorangehenden Ansprüche,
**dadurch gekennzeichnet,**
**dass** der Querschnitt der Zweige (7) rechteckförmig ist.

9. Vorrichtung nach einem der Ansprüche 1 bis 7,
**dadurch gekennzeichnet,**
**dass** der Querschnitt der Zweige die Form eines gleichschenkligen Trapez hat.

10. Vorrichtung nach einem der vorangehenden Ansprüche,
**dadurch gekennzeichnet,**
**dass** jeder Zweig ein Teil (30, 34) mit der Form einer Falte hat, die an der Seite der Spitze der Aufnahme liegt und eine Spitze (31) darstellt, die in das Innere der Aufnahme gerichtet ist.

11. Vorrichtung nach einem der vorangehenden Ansprüche,
**dadurch gekennzeichnet,**
**dass** der Stab (4) weich ist, das heißt durch Biegung unter einem normalen manuellen Druck durch einen Benutzer eine gekrümmte Form mit einer Durchbiegung von einem Zehntel bis einem Fünftel der Länge der Stange annehmen kann.

12. Vorrichtung nach einem der vorangehenden Ansprüche.
**dadurch gekennzeichnet,**
**dass** der Stab (4) einen mittleren Teil (10) für das Ergreifen durch die Finger des Benutzers enthält, der sich zu beiden Seiten der Symmetriemitte des Stabes erstreckt, mit einer leichten Neigung zu einer vorspringenden Erweiterung (11), die eine Anlage für die Finger des Benutzers bilden kann.
